# EUROPÄISCHE PATENTSCHRIFT

(11) **EP 0 241 811 B1**
(45) Veröffentlichungstag und Bekanntmachung des Hinweises auf die Patenterteilung: **01.12.1993**
(21) Anmeldenummer: 87104868.2
(22) Anmeldetag: 02.04.1987
(51) Int. Cl.: A61K 39/395, C07K 15/00, C12P 21/00, C12N 5/00, C12N 15/00

(54) **Monoclonale Antikörper auf human-Interleukin-2-Rezeptor**
Monoclonal antibodies against the human interleukin-2 receptor
Anticorps monoclonal contre le récepteur de l'interleukine-2 humaine

(30) Priorität: 14.04.1986 GB 8609058
(43) Veröffentlichungstag der Anmeldung: 21.10.1987
(73) Patentinhaber: BAYER AG, 51368 Leverkusen (DE)
(72) Erfinder: Diamantstein, Tibor, Prof. Dr., D-1000 Berlin 19 (DE); Osawa, Hisao, Dr., D-1000 Berlin 37 (DE)

(56) Entgegenhaltungen:
- EP-A- 0 171 496
- EP-A- 0 226 062
- EP-A- 0 235 805
- IMMUNOLOGICAL REVIEWS, Nr. 92, 1986, Munksgaard, Copenhagen (DK); T.DIAMANTSTEIN et al., Seiten 5-27#
- THE JOURNAL OF IMMUNOLOGY, Band 130, Nr. 1, Januar 1983, The American Association of Immunologists; H.OSAWA et al., Seiten 51-55#
- JOURNAL OF EXPERIMENTAL MEDICINE, Band 162, Juli 1985, The Rockefeller University Press; R.L.KIRKMAN et al., Seiten 358-362#
- MICROBIOL. IMMUNOL., Band 29, Nr. 10, 1985; Seiten 959-972#

## Beschreibung

Die Erfindung betrifft allgemein neue Hybrid-Zelllinien, und insbesondere Hybrid-Zellinien zur Herstellung monoclonaler Antikörper gegen ein Antigen, das auf aktivierten human-Lymphozyten gefunden wurde, den Interleukin-2-Rezeptor, die damit hergestellten Antikörper sowie therapeutische und diagnostische Methoden und Zubereitungen unter Verwendung der Antikörper.

### Einführung

In vielen Fällen, wenn nicht in allen, scheint der primäre Auslöser der Proliferation die Wechselwirkung von Wachstumsfaktoren mit dem Wachstumsfaktor-Rezeptor der Zelloberfläche zu sein. Die Aktivierung des Rezeptors des Wachstumsfaktors löst wiederum bisher ungeklärte zytoplasmische Signalsysteme aus.

Ruhende T-Lymphozyten stellen langlebige Zellen in der Gₒ-Phase des Zellzyklus dar. Diese treten in den Proliferationszyklus erst nach Antigen-Stimulierung in Gegenwart eines T-Zellen-Wachstumsfaktors, Interleukin-2 (IL-2) ein. Rezeptoren für IL-2 sind auf der Oberfläche ruhender T-Zellen nicht nachweisbar. Die Expression von IL-2-Rezeptoren (IL-2R) ist die Folge der Wechselwirkung von Antigen-präsentierenden Zellen mit dem Antigen-Rezeptor. Es konnte kürzlich gezeigt werden, dass die IL-2-Rezeptor-Expression ein Übergangsstadium darstellt, und daß eine wiederholte Restimulierung durch Lectine (Cantrell, P.A., und K.A. Smith, (1984), Science (Wash.D.C) 224:1312); (Osawa, H., und Diamantstein, T. (1984), J.Immunol. 132:2445) oder Antigen (Reske-Kunz, A.B., D.v. Steldern, E. Rüde, H. Osawa und T. Diamantstein, (1984), J. Immunol. 133:1356) zur kontinuierlichen IL-2-Rezeptor-Expression und folglich für ein langlebiges Zellwachstum erforderlich ist.

Da IL-2-R ausschliesslich auf aktivierten Lymphozyten exprimiert werden, können monoclonale Antikörper (mAb), die mit IL-2-R reagieren, als spezifisches und selektives immunsuppressives Agens geeignet sein. Ausserdem können solche Antikörper als diagnostische Reagenzien dienen, um qualitativ und quantitativ aktivierte Lymphozyten als auch neoplastische Zellen, die IL-2-R exprimieren, nachzuweisen.

### Zusammenfassung der Erfindung

Aufgabe der vorliegenden Erfindung ist es, eine Zubereitung aus mindestens zwei monoclonalen Antikörpern zur Verfügung zu stellen, die den human-Interleukin-2-Rezeptor erkennen und in der Lage sind, die Interleukin-2-induzierte Lymphozyten-Proliferation zu inhibieren. Nach einer bevorzugten Ausführungsform gehören einer oder mehrere der Antikörper der Klasse IgG₁ an. Nach einer weiteren bevorzugten Ausführungsform ist die Antikörper-Zubereitung in der Lage, die Interleukin-2-Bindung an den Rezeptor zu inhibieren. Die vorliegende Erfindung stellt monoclonale Antikörper der Klasse IgG₁ zur Verfügung, die human-Interleukin-2-Rezeptor erkennen, und in der Lage sind, die Interleukin-2-Bindung an den Rezeptor zu inhibieren und damit in der Lage sind, die Interleukin-2-abhängige Lymphozyten-proliferation zu inhibieren. Die Antikörper gemäß der vorliegenden Erfindung eignen sich zur Konstruktion chimärer tierischmenschlicher Antikörper gegen den menschlichen-Interleukin-2-Rezeptor, wobei die konstante Fc-Region des Immunoglobulins menschlichen Ursprungs und der variable Fab-Bereich tierischen Ursprungs ist. Bevorzugt wird der Fab-Bereich aus Mäusen erhalten.

Die vorliegende Erfindung stellt im weiteren Hybridoma-Zellinien zur Verfügung, die durch die Produktion/Sekretion monoclonaler Antikörper der Klasse IgG₁, welche den human-Interleukin-2-Rezeptor erkennen, charakterisiert sind. Besonders bevorzugt sind die Hybridoma-Zellinien der NTCC-Hinterlegungsnummer ECACC 86 04 1801 und ECACC 86 04 1802 (PHLS Centre of Applied Microbiology & Research, Porton Down, Salisbury, Wiltshire SP4 OJG, Great Britain).

Human-T-Lymphoblasten, welche IL-2-R exprimieren, wurden nach an sich bekannten Methoden hergestellt (Osawa, H., und Diamantstein, T. (1983) J. Immunol. 130:51) und wurden eingesetzt, um monoclonalen Maus-Antikörper gegen IL-2-R nach dem Verfahren von Köhler und Milstein (Köhler, G., und C. Milstein, (1975) Nature 256:495) herzustellen. Die Fusion ergab zwei Hybrid-Clone, AHT-54 und AHT-107, welche Anti-IL-2-R-Antikörper der Unterklasse IgG₁ produzieren. Die Hybridclone, die Anti-IL-2-R-Antikörper sekretieren, wurden als bevorzugte Ausführungsform gemäß der Erfindung selektiert.

Beide mAb i) inhibieren die Bindung von ¹²⁵I-markiertem IL-2 an IL-2-R-positive human-Lymphozyten, ii) inhibieren die IL-2-abhängige Proliferation in vitro, und iii) präzipitieren das identische Zelloberflächenmolekül von 55 KD, das IL-2-Bindungsprotein. Die kompetitive Bindung von AHT-54 und AHT-107 zeigte, dass sie unterschiedliche Epitope des IL-2-R-Moleküls erkennen.

AHT-107 unterscheidet sich von Anti-Tac (Uchiyama, T. et al., (1981) J. Immunol. 126:1398), da kompetitive Inhibierungsstudien zeigten, dass sie zwei unterschiedliche Epitope des IL-2-R-Moleküls erkennen; AHT-107 unterscheidet sich auch von 7G7 B6, einem kürzlich beschriebener Anti-human-IL-2-R mAb (A. Rubin, C. Kurman, E. Biddison, D. Goldman und L. Nelson (1985) Hybridoma Vol. 4:91), da im Gegensatz zu 7G7 B6 AHT-107 die Bindung von IL-2 an den IL-2-R wie auch die IL-2-abhängige Proliferation von Lymphozyten inhibiert.

Desgleichen unterscheiden sich die hier beschriebenen Antikörper von dem in TANAKA et al., Microbiol. Immunol. Vol. 29 (10), 959-972 (1972) beschriebenen monoklonalen Antikörper.

EP-A-0 235 805 beschreibt ein Cocktail aus ähnlichen anti T-cell-Antikörpern.

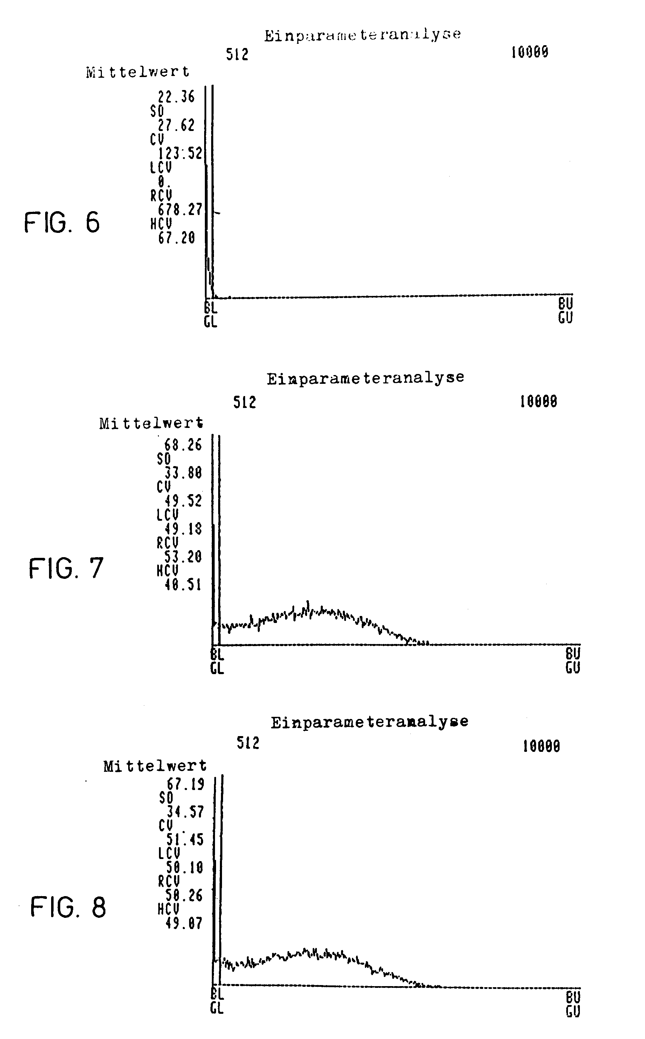

Beide mAb reagieren spezifisch mit aktivierten Lymphozyten (T und B), jedoch nicht mit ruhenden Lymphozyten oder anderen nicht-lymphoiden Zellen. Diese Behauptung basiert auf FACS-Analysedaten (Fig. 6 bis 11).

Gemäß früheren Untersuchungen an Tiermodellen wurde gezeigt, daß solche Ab, welche mit Ratten (ART-18) und mit Mäuse-IL-2-R (AMT-13 und M7/20) reagieren, selektiv und spezifisch inhibieren: i) die lokale GVH-Reaktion (Diamantstein, T. und H. Osawa, (1986), Immune Rev. 92, in Druck), ii) die Cardio-Allotransplantat-Abstossung (L. Kirkman, E. Kelley, A. Koltun, J. Schoen, A. Ythier und B. Strom, (1985), Transplantation 40:719), (L. Kirkman, L.V. Barett, N. Gaulton,
E. Kelley, A. Ythier und B. Strom, (1985), J. Exp. Med. 162; 358), und iii) die T-Zell-vermittelte Autoimmunreaktion, wie z.B. akute Autoimmun-Encephalomyelitis und Adjuvans-Arthritis, welche durch T-Zell-Übertragung induziert wurden (Wekerle, H. und T. Diamantstein, (1986), Autoimmunity: Experimental and Clinical Aspects, Hsg: R.S. Schwarz, N.R. Rose, Ann. New York Acad. Sci., in Druck).

Die monoclonalen Anti-IL-2-R-Antikörper gemäss der Erfindung eignen sich auch als therapeutische Agenzien für klinische Syndrome, die im Zusammenhang mit der pathologischen Proliferation von IL-2-abhängigen Zellen stehen. So können z.B. Hyperimmun-Syndrome, wie Wirt-gegen-Transplantat (HvG)-, Transplantat-gegen-Wirt (GvH)-Erkrankungen und Autoimmun-Erkrankungen (z.B. Multiplis Sclerose, Autoimmun-Diabetes, Erkrankungen nach Crohn) behandelt werden. Nach einer bevorzugten Ausführungsform gemäss der vorliegenden Erfindung werden monoclonale Anti-IL-2-R-Antikörper direkt als therapeutische Agenzien, ohne weitere Modifizierung derselben, verwendet. Ausserdem umfaßt die Erfindung eine Zubereitung von chimären Anti-IL-2-R-Antikörpern unter Verwendung der schweren human-Kette verschiedener Klassen und Unterklassen in Kombination mit der variablen Region des AHT-54 und AHT-107 mAb, um die therapeutische Anwendung zu optimieren.

Alternativ können die Antikörper an Arzneimittel, einschliesslich cytotoxischer Agenzien, gekoppelt werden. Die monoclonalen Antikörper gemäss der Erfindung sind geeignet, Antigen-aktivierte Zellen, die IL-2-Rezeptoren exprimieren, zu erkennen, deren Funktion zu inhibieren und diese selektiv zu eliminieren.

Die monoclonalen Antikörper der vorliegenden Erfindung eignen sich auch als diagnostische Reagenzien auf Zellen, die IL-2-R entweder auf der Zelloberfläche oder innerhalb der Zellen oder in Körperflüssigkeiten enthalten. Somit ist es mit Hilfe der Erfindung möglich, Zellen, die IL-2-R enthalten, in Proben mit verschiedenen Zellarten zu identifizieren. Die Lokalisierung von IL-2-haltigen Zellen ist in den Kulturzellkolonien oder in Gewebeproben möglich. Bei einer derartigen Anwendung werden die monoclonalen Antikörper vorzugsweise an fluoreszierende, farbbildende Substanzen, wie z.B. ein Enzym oder ein Chromophor oder eine radioaktive Substanz (ELISA, RIA) gekoppelt.

### I. Herstellung von mAb

### Quelle für IL-2-R

IL-2-R-exprimierende Zellen wurden, wie beschrieben, unter Verwendung von human-T-Lymphoblasten hergestellt. Gemischte periphere human Blut-Lymphozyten wurden mit 3 mg/ml Concanavalin A (Con A) 3 Tage lang stimuliert. Die konvertierten Zellen wurden mit alpha-Methylmannosid (20 mg pro ml) behandelt, gewaschen und als Immunogen in Kulturmedium verwendet. Die Kulturen wurden in Click's RPMI-Medium (Seromed GmbH, München, BRD) angesetzt, welches mit 2 x 10⁻³M L-Glutamin, 5 x 10⁻⁵ M 2-Mercaptoethanol, 100 E/ml Penicillin, 100 µg/ml Streptomycin, und 5 bis 10 % (V/V) Fötal-Kälberserum (FCS; Charge Nr. 104; Seromed GmbH) ergänzt war.

### Immunisierung, Zellfusion, Clonierung und Gewinnung von monoclonalen Antikörpern (mAb)

10 Wochen alte BALB/c-Mäuse wurden mit 2 x 10⁷ T-Lymphoblasten geprimt. Die Zellen wurden in 0,1 ml Anteilen (10⁶ Zellen) subkutan in das Fusspolster und den Nacken der Mäuse wie auch i.v. (10⁷ Zellen in 0,5 ml) injiziert. 4 Wochen darauf erhielten die Mäuse i.v. 10⁷ T-Lymphoblasten. 3 Tage darauf wurden Milzzellen der immunisierten Mäuse mit X63-Ag8.653 Maus-Myelomazellen in Gegenwart von Polyethylenglykol fusioniert (Köhler und Milstein, (1975), Nature 256:495, modifiziert durch Lemke H., G.J. Hämmerling, C. Höhmann und K. Rajewsky, (1978), Nature 271:249). Fusionierte, in HAT-Medium suspendierte Zellen wurden in jede Vertiefung von 10 Gewebekulturplatten mit jeweils 24 Vertiefungen verteilt (1 bis 2 x 10⁶ Milzzellen/Vertiefung). Die Überstände in den Vertiefungen, in denen nach 3 bis 4 Wochen ein kräftiges Wachstum beobachtet wurde, wurden untersucht im Hinblick auf ihre Kapazität an a) human-T-Lymphoblasten, b) Maus-T-Lymphoblasten und c) human-Thymozyten, die an der Oberfläche der Vertiefungen von Mikrotiter-Platten hafteten, zu binden. Zell-gebundenes Immunoglobin wurde dann mit Hilfe eines Enzym-Immunosorbent-Assays (ELISA), wie beschrieben, nachgewiesen (Kincade, P.W., G. Lee, L. Sun, und T. Watanabe, (1981), J. Immunol. Methods 42:17) unter Verwendung von β-Galactosidasegekoppeltem Schaf-F(ab')₂-anti-Maus Immunoglobulin (New England Nuclear, Dreieich, BRD) als zweiten Antikörper. Die Hybridomzellen, die in HAT- oder RPMI-Medium gezüchtet wurden und konstant Antikörper produzierten, die spezifisch an human-T-Lymphoblasten banden, wurden selektiert. Die Überstände der wachsenden Hybridomazellen wurden wiederholt getestet und im Hinblick auf Hybridomazellen selektiert, die Überstände produzierten, welche im funktionellen Assay (Inhibierung der T-Lymphoblasten-Antwort auf IL-2) wie auch beim Absorptions-Assay (Inhibierung der Fähigkeit der T-Lymphoblasten, IL-2 nach Preinkubation zu absorbieren) aktiv waren. Positive Hybridome wurden mittels beschränkter Verdünnung mit Maus-Thymozyten, die als Feeder-Schicht verwendet wurden, geclont. Die Clone wurden erneut getestet und ausgestrichen. Die Überstände der relevanten Clone wurden zur Isolierung und Reinigung von mAb verwendet.

### Reinigung von mAb

Tests, die mittels der Ouchterlony-Doppel-Immunodiffusions-Methode mit Kaninchen-anti-Maus-IgM-, IgA-, IgG₁-, IgG₂ₐ-, IgG_{2b}- und IgG₃-Seren (Miles Laboratories, Ltd., Slough, England) durchgeführt wurden, zeigten, daß die Hybridoma AHT-54 und AHT-107 IgG₁-Antikörper sezernieren. Ausgenommen der ursprünglichen Screening-Experimente, in denen nicht-gereinigte Kultur-Überstände verwendet wurden, wurden die folgenden Experimente mit gereinigtem IgG₁ durchgeführt. Die Reinigung wurde durch sukzessive Bindung/Elution von Protein A-Sepharose (Pharmacia Fine Chemicals) gemäss dem Verfahren von Ey et al ( Ey, P.L., S.J. Prowse und C.R. Jenkin, (1978), Immunochemistry 15:429) erzielt. Ca. 600 ml der Kultur-Überstände, die auf pH 8,0 gebracht worden waren, wurden über eine 5ml Protein-A-Sepharose-Säule gegeben, welche mit 0,1 M Natriumphosphatpuffer (pH 8,0) equilibriert war. IgG₁ wurde von der Säule mit Hilfe von 0,1 M Natriumzitratpuffer (pH 6,0) eluiert. Der gereinigte Antikörper wurde dann gegen einen Puffer dialysiert, der 0,01 M HEPES (pH 7,4) und 0,9 % NaCl enthielt. Die Reinheit von mAb wurde mittels Natriumdodecylsulfat (SDS)-Polyacrylamid-Gelelektrophorese bestätigt, welche unter reduzierenden Bedingungen durchgeführt wurde, wie dies in der Literatur beschrieben ist (Laemmli, GB 1970, Cleavage of structural proteins during the assembly of the head of bacteriophage T₄, Nature 227:429). Die Proteinkonzentraton des gereinigten IgG₁ wurde durch Absorption von ultraviolettem Licht bei 280 nm bestimmt, wobei ein Extinktionskoeffizient (1% G/V:1cm) von 14 angenommen wurde und nach dem Verfahren von Lowry et al, 1951 (Lowry, O.H., N.J. Rosebrogh, A.L. Farr, und R.J. Randall (1951)), wobei Rinderserumalbumin (BSA) als Standard verwendet wurde.

Rekombinantes Interleukin-2 wurde von Sanolez Mena erhalten. ¹²⁵I-markiertes rekombinantes IL-2 wurde von NEN bezogen.

### Markierung von mAb mit ¹²⁵I

MoAb wurden mit ¹²⁵I gemäß MacConahey und Dixon (McConahey, P.J., und F.J. Dixon, (1980) Methods Enzymol, 70:210) markiert. Kurzgesagt: 20 µg IgG₁, aufgelöst in 60 µl Na¹²⁵J (100 mCi pro ml, trägerfrei; Amersham Buchler). 10 µl Chloramin-T (2,5 mg/ml in 0,05 M Na-P) wurden zu dem Gemisch zugegeben. Nach 45 Sekunden Inkubation bei Raumtemperatur wurden der Lösung 20 µl Na₂S₂O₅ (3mg/ml in 0,05 Na-P) einzugeben. Das Gemisch wurde sofort auf eine 15 ml Sephadex™-G-75-Säule aufgetragen (vorgewaschen mit 0,05 M Na-P, welcher 4 % BSA enthielt, und nacheinander gewaschen mit 0,05 M Na-P, bis das Eluat proteinfrei war) und die Radiomarkierung in der ausgeschlossenen Fraktion gesammelt.

### Detaillierte Beschreibung der Zeichnungen:

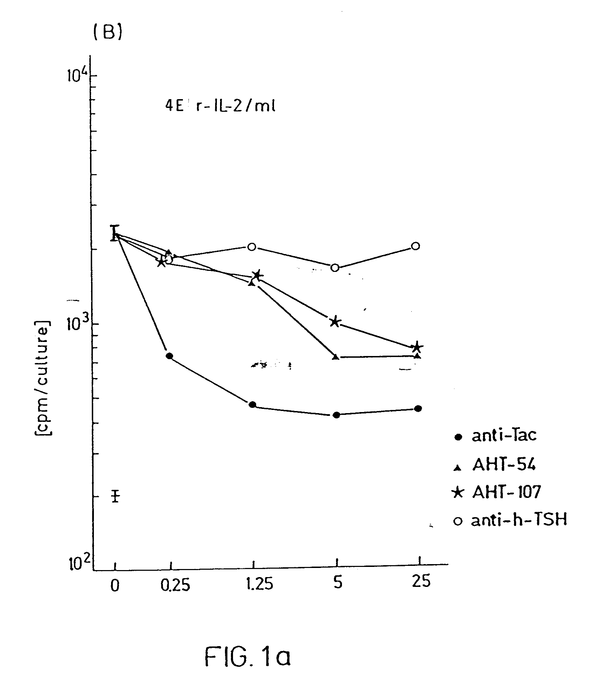

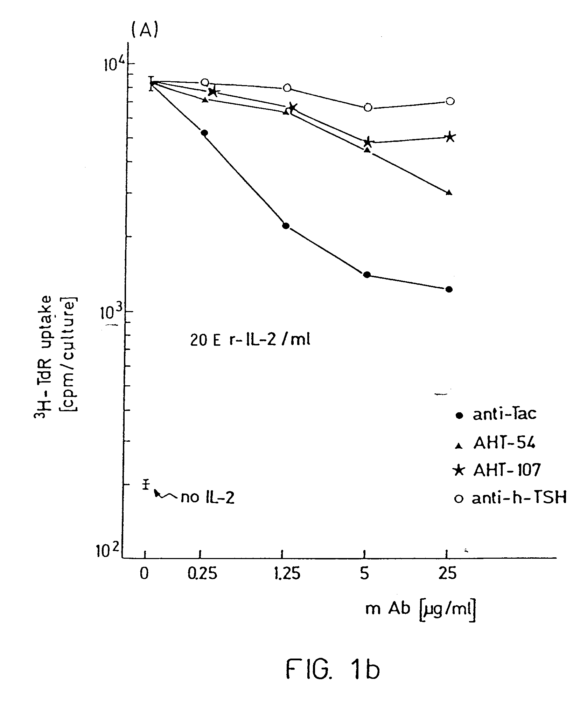

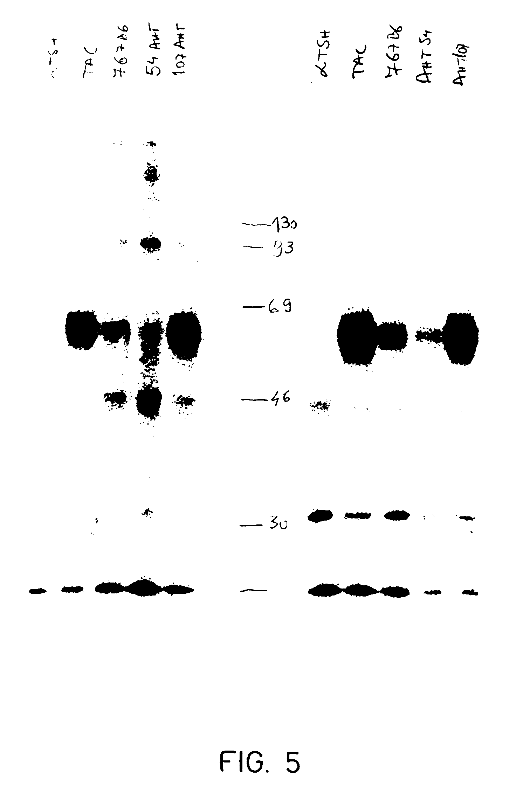

Fig. 1 Inhibierung von IL-2-abhängiger human-T-Lymphoblasten-Proliferation durch verschiedene mAbs
   2 x 10⁶ human-T-Lymphoblasten wurden 3 Tage lang in 0,2 ml Medium inkubiert, welches die angegebenen Mengen an rekombinantem IL-2 (Fig. 1b, 20 E/ml; Fig. 1b, 4E/ml) in Abwesenheit oder Gegenwart verschiedener mAbs, anti-Tac (-o-), AHT-54(-Δ-), AHT-107 (-*-) und einem Kontroll-mAb, anti-human-TSH (-o-), enthielt. Die Zellen wurden mit ³H-Thymidin während der letzten 4 h der Inkubationsdauer pulsiert. Die Inkorporierung von ³H-Thymidin wurde nach dem Standard-Verfahren gemessen (Diamantstein et al, Mol. Immunol., (1984), 21:1229).

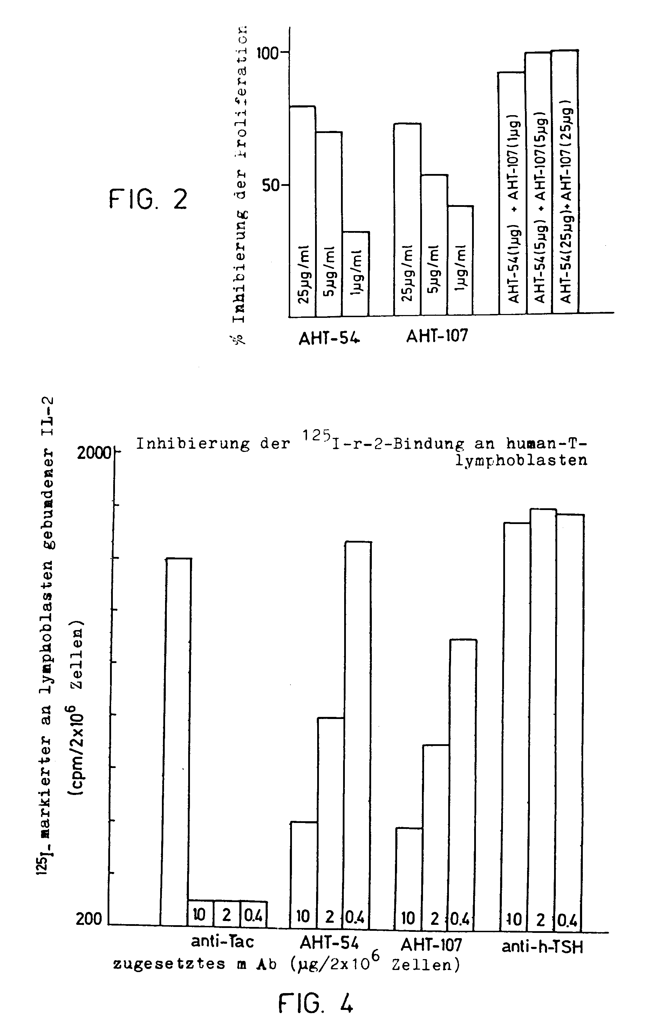

Fig. 2 Synergistische Wirkung von AHT-54 und AHT-107 mAbs auf die IL-2-abhängige Proliferation
   T-Lymphoblasten wurden mit 20E/ml r-IL-2 drei Tage lang (wegen Details siehe Fig. 1) in Gegenwart von entweder AHT-54 oder AHT-107 mAb oder in Kombination beider mAbs kultiviert.

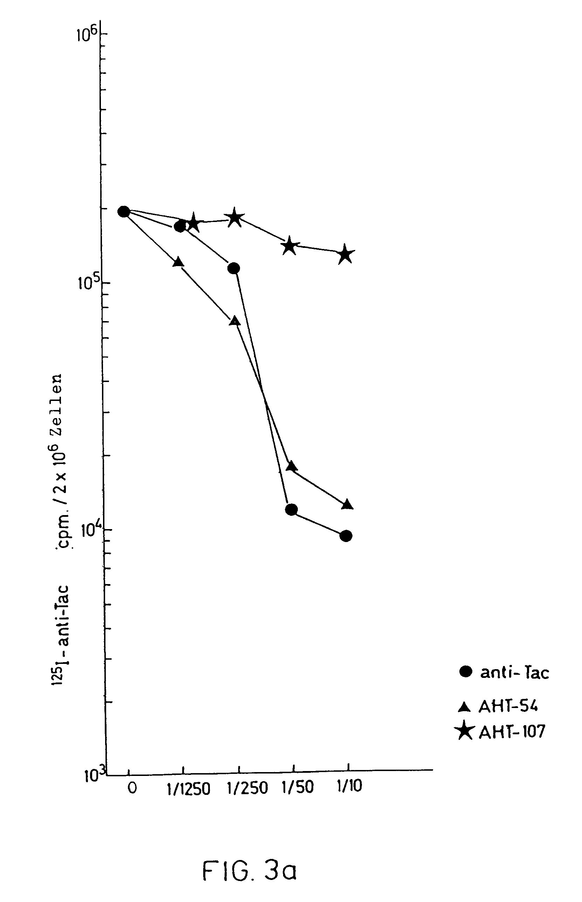

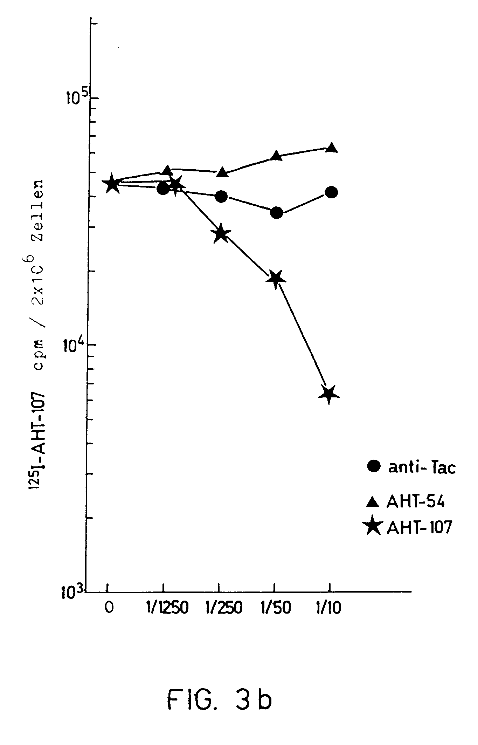

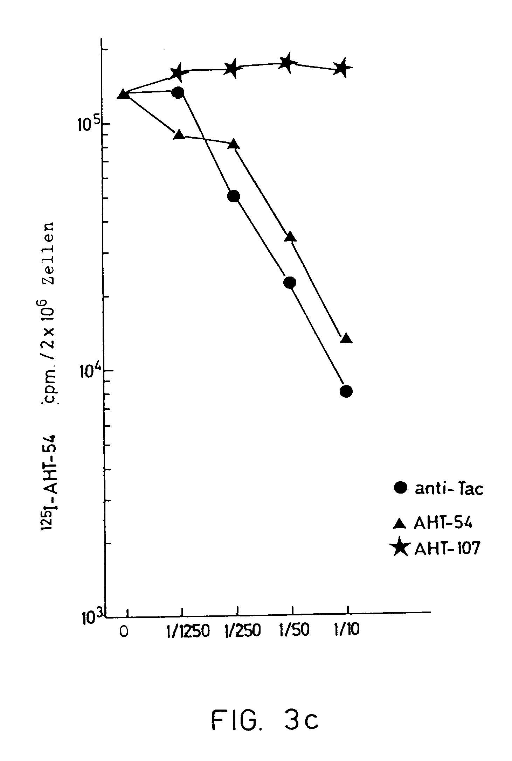

Fig. 3 Kompetition um die Bindung von ¹²⁵I-markierten Antikörpern
   2 x 10⁶ human-T-Blasten wurden zunächst in 100 µl eines Bindungspuffers suspendiert (PBS=0,5% BSA/10 mM NaN₃), welcher verschiedene Verdünnungen der mAbs-anti-Tac (-o-), AHT-54 (-Δ-) und AHT-107 (-*-) enthielt. Die Suspensionen wurden mit 100 µl einer 1:40-Verdünnung des ¹²⁵I-markierten mAb gemischt.
   Das Gemisch wurde 1 h bei 4°C inkubiert. Die relative Menge an ¹²⁵I-markiertem mAb (cpm), welche an die pelletierten Zellen gebunden war, wurde nach zwei Waschgängen mit dem Bindungspuffer unter Verwendung eines Gamma-Strahlen-Zählers gemessen.
Fig. 4 Inhibierung der ¹²⁵I-IL-2-Bindung an human-T-Blasten durch verscheidene mAbs
   2 x 10⁶ human-T-Blasten wurden zunächst 30 Minuten bei 37°C 0,25 ml eines Puffers inkubiert (RPMI/Hepes/BSA/NaN₃), welcher die angegebenen Mengen verschiedener mAbs enthielt. Die Inkubation wurde in Gegenwart von ¹²⁵I-IL-2 bei 37°C fortgesetzt. Nach 40 Minuten wurde das Inkubationsgemisch zentrifugiert, um die Zellen zu pelletieren; die pelletierten Zellen wurden in 100 µl des Puffers aufgenommen und einer Ölphase überlagert, die aus Dibutylphthalat/Olivenöl (10+3) bestand. Nach Zentrifugation wurden die Reagenzglasspitzen, die die Zellpellets enthielten, abgetrennt und in einem gamma-Strahlen-Zähler ausgezählt.
Fig. 5 SDS-PAGE-Analyse von Immunoprecipitaten mit verschiedenen mAbs
   2 x 10⁷ human-T-Blasten wurden mit 0,5 mCi Na[¹²⁵I] oberflächenjodiert und in 0,5 ml des Lysepuffers lysiert. Das Lysat wurde zentrifugiert und mit 1/5 Volumen an Protein-A-Sepharose™-Kügelchen(10 µl) über Kaninchen-anti-Maus-IgG als Brückenantikörper preabsorbiert. Nach 1 h bei 4°C wurden die Kügelchen dreimal mit Puffer gewaschen, welcher 50 mM tris-HCl, pH 8,3, 450 mM NaCl, 5 mM KI, 0,02 % NaN₃ und 0,5 Nonidet™ P 40 enthielt, und mit 100 µl des Probepuffers extrahiert. 50 µl aliquote Teile des Extraktes wurden einer SDS-PAGE-Analyse unterworfen, und zwar entweder unter nicht-reduzierenden (Bahnen 1 bis 6) oder reduzierenden (Bahnen 7 bis 12) Bedingungen. Die verwendeten mAbs waren Maus-UPC-10-Ascites als Kontrolle (Bahnen 1 & 7), anti-Tac-Ascites (Bahnen 2 & 8), AHT-54-Ascites (Bahnen 4 und 10), AHT-107-Ascites (Bahnen 5 & 11), und AHT-107-Kulturüberstand (Bahnen 6 & 12).
Fig. 6 bis 11 FACS-Analyse
   Humane periphere Blutzellen (HPBL) und aktivierte T-Lymphoblasten, die von HPBL abgeleitet waren, wurden bei 4°C in Gegenwart von 0,1 % NaN₃ 30 Minuten mit AHT-54 oder AHT-107 mAb inkubiert (1:1000 Ascites-Flüssigkeit) und als negataive Kontrolle mit einem TSH, gewaschen und angefärbt unter Verwendung einer sättigenden Menge an Ziegen-anti-Maus-IgG, markiert mit FITC. Fluoreszenz-aktivierte Zell Sorter-Analysen wurden mit einem Epics V vorgenommen.

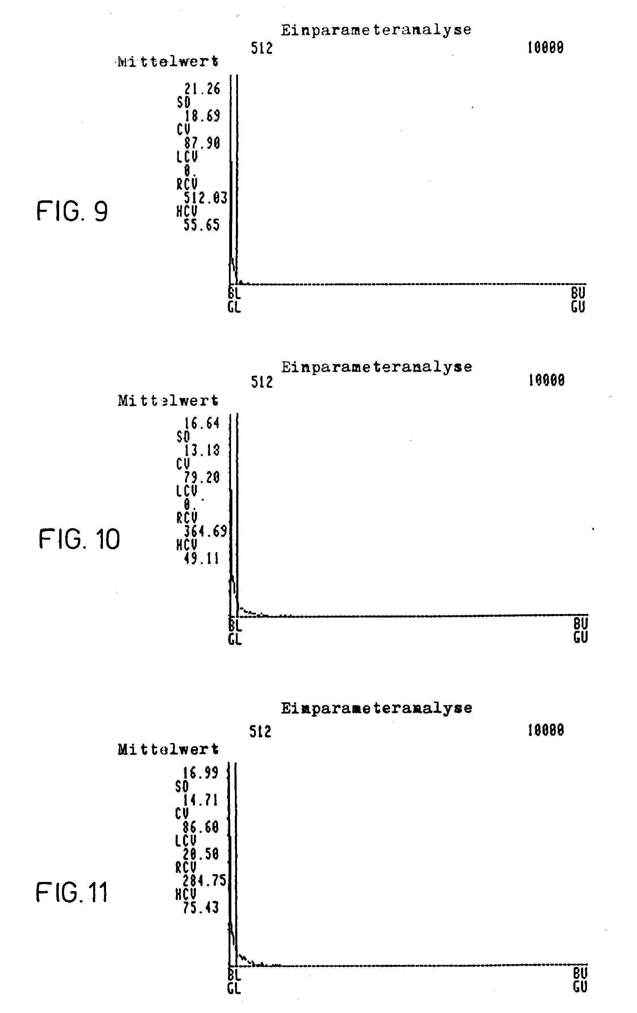

   Fig. 6 zeigt die negative Kontrolle. Der alpha-TSH-Antikörper band nicht an die T-Lymphoblasten. In Fig. 7 und 8 wird gezeigt, dass AHT-54 (Fig. 7) und AHT-107 (Fig. 8) in der Lage sind, an Lymphoblasten zu binden. Fig. 9 bis 11 beziehen sich auf das gleiche Experiment, mit dem Unterschied, daß die Lymphoblasten gegen HPBL ausgetauscht wurden. Bei keinem der drei Antikörper tritt eine Reaktion auf.

## Patentansprüche (Patentansprüche für folgende(n) Vertragsstaat(en): AT, BE, CH, DE, FR, GB, IR, IT, LI, NL, SE)

1. Zubereitung aus mindestens zwei monoclonalen Antikörpern, welche den human-Interleukin-2-Rezeptor erkennen und in der Lage sind, die Interleukin-2-abhängige Lymphozyten-Proliferation zu inhibieren.

2. Zubereitung nach Anspruch 1, gekennzeichnet durch die Inhibierung der Interleukin-2-Bindung an den Rezeptor.

3. Hybridoma-Zellinien, gekennzeichnet durch die Herstellung von monoclonalen Antikörpern, mit der NTCC-Hinterlegungs-Nummer ECACC 86 04 1801 und ECACC 86 04 1802.

4. Chimärer tierisch-menschlicher Antikörper, welcher den human-Interleukin-2-Rezeptor erkennt.

5. Chimärer Antikörper nach Anspruch 4, dadurch gekennzeichnet, daß der Fc-Anteil menschlichen Ursprungs und der variable Teil tierischen Ursprungs ist.

6. Chimärer Antikörper nach Ansprüchen 4 und 5, dadurch gekennzeichnet, daß die Fab-Region von Mäusen stammt.

7. Chimärer Antikörper nach den Ansprüchen 4 bis 6, dadurch gekennzeichnet, daß der variable Teil von den monoclonalen Antikörpern AHT-54 oder AHT-107 stammt.

8. Chimärer Antikörper nach den Ansprüchen 4 bis 7 zur Verwendung in einem Verfahren zur therapeutischen und/oder diagnostischen Behandlung des menschlichen Körpers.

9. Chimärer Antikörper nach den Ansprüchen 4 bis 8 zur Verwendung in einem Verfahren zur Therapie und/oder Diagnose klinischer Syndrome, die im Zusammenhang mit der pathologischen Proliferation von Interleukin-2-abhängigen Zellen stehen.

10. Arzneimittel enthaltend einen chimären tierisch-menschlichen Antikörper, der den humanen Interleukin-2-Rezeptor erkennt.

## Patentansprüche (Patentansprüche für folgende(n) Vertragsstaat(en): ES)

1. Verwendung chimärer tierisch-menschlicher Antikörper, welche den humanen Interleukin-2-Rezeptor erkennen, zur Herstellung eines Arzneimittels zur therapeutischen und diagnostischen Behandlung klinischer Syndrome, die im Zusammenhang mit der pathologischen Proliferation von Interleukin-2-abhängigen Zellen stehen.

2. Verwendung chimärer tierisch-menschlicher Antikörper nach Anspruch 1, dadurch gekennzeichnet, daß der Fc-Anteil menschlichen Ursprungs und der variable Teil tierischen Ursprungs ist.

3. Verwendung chimärer tierisch-menschlicher Antikörper nach Anspruch 1, dadurch gekennzeichnet, daß die Fab-Region von Mäusen stammt.

4. Verwendung chimärer tierisch-menschlicher Antikörper nach Anspruch 1, dadurch gekennzeichnet, daß die Antikörper aus monoklonalen Antikörpern mit den NTCC-Hinterlegungsnummern ECACC 86041801 und ECACC 86041802 abgeleitet sind.

5. Verwendung chimärer tierisch-menschlicher Antikörper nach Anspruch 4, dadurch gekennzeichnet, daß der variable Teil von den monoklonalen Antikörpern AHT-54 oder AHT-107 stammt.

## Claims (Claims for the following Contracting State(s): AT, BE, CH, DE, FR, GB, IT, LI, NL, SE)

1. Preparation consisting of at least two monoclonal antibodies which recognise the human interleukin-2 receptor and are able to inhibit interleukin-2-dependent lymphocyte proliferation.

2. Preparation according to Claim 1, characterised by the inhibition of interleukin-2 binding to the receptor.

3. Hybridoma cell lines, characterised by the production of monoclonal antibodies having the NTCC deposition numbers ECACC 86 04 1801 and ECACC 86 04 1802.

4. Chimeric animal-human antibody which recognises the human interleukin-2 receptor.

5. Chimeric antibody according to Claim 4, characterised in that the Fc moiety is of human origin and the variable part is of animal origin.

6. Chimeric antibody according to Claims 4 and 5, characterised in that the Fab region is derived from mice.

7. Chimeric antibody according to Claims 4 to 6, characterised in that the variable part is derived from the monoclonal antibodies AHT-54 or AHT-107.

8. Chimeric antibody according to Claims 4 to 7 for use in a process for the therapeutic and/or diagnostic treatment of the human body.

9. Chimeric antibody according to Claims 4 to 8 for use in a process for the therapy and/or diagnosis of clinical syndromes which are connected with the pathological proliferation of interleukin-2-dependent cells.

10. Medicament containing a chimeric animal-human antibody which recognises the human interleukin-2 receptor.

## Claims (Claims for the following Contracting State(s): ES)

1. Use of chimeric animal-human antibodies, which recognise the human interleukin-2 receptor, for preparing a medicament for the therapeutic and diagnostic treatment of clinical syndromes which are connected with the pathological proliferation of interleukin-2-dependent cells.

2. Use of chimeric animal-human antibodies according to Claim 1, characterised in that the Fc moiety is of human origin and the variable part is of animal origin.

3. Use of chimeric animal-human antibodies according to Claim 1, characterised in that the Fab region is derived from mice.

4. Use of chimeric animal-human antibodies according to Claim 1, characterised in that the antibodies are derived from monoclonal antibodies having the NTCC deposition numbers ECACC 86041801 and ECACC 86041802.

5. Use of chimeric animal-human antibodies according to Claim 4, characterised in that the variable part is derived from the monoclonal antibodies AHT-54 or AHT-107.

## Revendications (Revendications pour l'(les) Etat(s) contractant(s) suivant(s): AT, BE, CH, DE, FR, GB, IT, LI, NL, SE)

1. Préparation comprenant au moins deux anticorps monoclonaux qui reconnaissent le récepteur de l'interleukine 2 humaine et qui sont en mesure d'inhiber la prolifération des lymphocytes dépendant de l'interleukine 2.

2. Préparation selon la revendication 1, caractérisée par l'inhibition de la liaison de l'interleukine 2 au récepteur.

3. Lignées cellulaires d'hybridomes, caractérisées par la mise au point d'anticorps monoclonaux dont les numéros de dépôt NTCC sont ECACC 86 04 1801 et ECACC 86 04 1802.

4. Anticorps chimère animal-humain qui reconnaît le récepteur de l'interleukine 2 humaine.

5. Anticorps chimère selon la revendication 4, caractérisé en ce que la fraction Fc est d'origine humaine et la fraction variable est d'origine animale.

6. Anticorps chimère selon les revendications 4 et 5, caractérisé en ce que la région Fab provient de souris.

7. Anticorps chimère selon les revendications 4 à 6, caractérisé en ce que la partie variable provient des anticorps monoclonaux AHT-54 ou AHT-107.

8. Anticorps chimère selon les revendications 4 à 7, pour être utilisé dans un procédé pour le traitement thérapeutique et/ou diagnostique du corps humain.

9. Anticorps chimère selon les revendications 4 à 8, pour être utilisé dans un procédé pour la thérapie et/ou le diagnostic de syndromes cliniques qui ont un rapport avec la prolifération pathologique de cellules dépendantes de l'interleukine 2.

10. Médicament contenant un anticorps chimère animal-humain qui reconnaît le récepteur de l'interleukine 2 humaine.

## Revendications (Revendications pour l'(les) Etat(s) contractant(s) suivant(s): ES)

1. Utilisation d'anticorps chimères animaux-humains qui reconnaissent le récepteur de l'interleukine 2 humaine pour la préparation d'un médicament destiné au traitement thérapeutique et diagnostique de syndromes cliniques qui ont un rapport avec la prolifération pathologique de cellules dépendantes de l'interleukine 2.

2. Utilisation d'anticorps chimères animaux-humains selon la revendication 1, caractérisée en ce que la fraction Fc est d'origine humaine et la fraction variable est d'origine animale.

3. Utilisation d'anticorps chimères animaux-humains selon la revendication 1, caractérisée en ce que la région Fab provient de souris.

4. Utilisation d'anticorps chimères animaux-humains selon la revendication 1, caractérisée en ce que les anticorps dérivent d'anticorps monoclonaux dont les numéros de dépôt NTCC sont ECACC 86 04 1801 et ECACC 86 04 1802.

5. Utilisation d'anticorps chimères animaux-humains selon la revendication 4, caractérisée en ce que la partie variable provient des anticorps monoclonaux AHT-54 ou AHT-107.
